(19) Europäisches Patentamt
European Patent Office
Office européen des brevets

(11) EP 4 659 735 A1

(12) # EUROPEAN PATENT APPLICATION
published in accordance with Art. 153(4) EPC

(43) Date of publication:
**10.12.2025 Bulletin 2025/50**

(21) Application number: **24750203.2**

(22) Date of filing: **29.01.2024**

(51) International Patent Classification (IPC):
***A61K 8/49** (2006.01)* ***A61K 8/22** (2006.01)*
***A61K 8/41** (2006.01)* ***A61Q 5/06** (2006.01)*

(52) Cooperative Patent Classification (CPC):
**A61K 8/22; A61K 8/41; A61K 8/49; A61Q 5/06; A61Q 5/10**

(86) International application number:
**PCT/JP2024/002635**

(87) International publication number:
**WO 2024/162254 (08.08.2024 Gazette 2024/32)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **30.01.2023 JP 2023011708**

(71) Applicant: **KAO CORPORATION**
**Tokyo 103-8210 (JP)**

(72) Inventors:
- **SUGIURA Kosaku**
**Tokyo 131-8501 (JP)**
- **NAGAYAMA Ayami**
**Tokyo 131-8501 (JP)**

(74) Representative: **Hoffmann Eitle**
**Patent- und Rechtsanwälte PartmbB**
**Arabellastraße 30**
**81925 München (DE)**

(54) **HAIR TREATMENT METHOD**

(57) A hair treatment method comprising in order the following step (I) and step (II): (I) applying a hair dye composition A comprising the following component (A1) and component (A2) to hair to dye the hair: (A1) an oxidation dye, and (A2) hydrogen peroxide; and (II) applying a composition B comprising the following component (B1) and component (B2) and having pH of 8.0 or higher and 11.0 or lower to the hair: (B1) a certain melanin precursor or a salt thereof, and (B2) an alkali agent.

EP 4 659 735 A1

## Description

Field of the Invention

The present invention relates to a hair treatment method.

Background of the Invention

Hair coloring requires long process time and causes hair damage. Therefore, a method for improving color retention of hair after dyeing is demanded for decreasing the number of times of hair dyeing. Specifically, a hair treatment method which offers less discoloration is demanded even if hair wash is performed after hair dyeing.

Various methods employing a pretreatment agent or an aftertreatment agent of hair dyeing are under study on the method for improving color retention of hair.

For example, JP-A-2015-140326 (Patent Literature 1) discloses that a method for preventing color fading of dyed hair, characterized by coating the hair with an anti-fading agent containing a certain lactone derivative, and subjecting the hair to heat treatment can exert an excellent anti-fading effect on the dyed hair.

JP-A-2005-170838 (Patent Literature 2) discloses that a pretreatment agent composition for dyeing hair, characterized by containing a direct dye, glycine and/or taurine, and water can provide excellent uniform dyeability and robustness to hair having various hair dyeing histories.

JP-A-2005-320355 (Patent Literature 3) recites that a an aftertreatment agent for dyeing hair composed of a pH buffering agent composed of an organic acid and/or an organic acid salt and an alkali agent, and a wheat protein hydrolysate and/or a silylated protein hydrolysate, when applied to hair treated by hair dyeing, can improve texture and color retention while repairing damage ascribable to hair dyeing and preventing accumulation of damage.

JP-A-2008-133227 (Patent Literature 4) discloses a two-part hair treatment agent for use in pretreatment prior to hair dyeing treatment with an oxidation-type hair dye, the two-part hair treatment agent being composed of a first part for coating hair first and a second part for coating the hair following the first part, wherein the first part contains a keratin derivative, hydrolyzed keratin, or a derivative thereof, and the second part contains amino-modified silicone, and Patent Literature 4 states that this two-part hair treatment agent can enhance uniform dyeability during hair dyeing and color robustness after hair dyeing.

JP-A-2021-59517 (Patent Literature 5) discloses that a hair cosmetic containing at least one component selected from the group consisting of hydroxypropyl gluconamide and hydroxypropyl ammonium gluconate, and at least one component selected from the group consisting of glutamic acid, aspartic acid, and acetylhydroxyproline in their respective certain amounts improves the elasticity of damaged hair, offers excellent moist feeling and smooth texture, and is excellent in effect of preventing hair color fading (color loss).

JP-A-2021-123535 (Patent Literature 6) discloses that an anti-fading composition for dyeing hair, containing at least one first compound selected from the group consisting of a certain L-ascorbic acid derivative and a salt thereof, and a cationic second compound is capable of reducing or suppressing color fading of hair after hair dyeing.

Summary of the Invention

The present invention relates to the following.

[1] A hair treatment method comprising in order the following step (I) and step (II):

(I) applying a hair dye composition A comprising the following component (A1) and component (A2) to hair to dye the hair:

(A1) an oxidation dye, and
(A2) hydrogen peroxide; and

(II) applying a composition B comprising the following component (B1) and component (B2) and having pH of 8.0 or higher and 11.0 or lower to the hair:

(B1) a compound of the following formula (1) or a salt thereof:

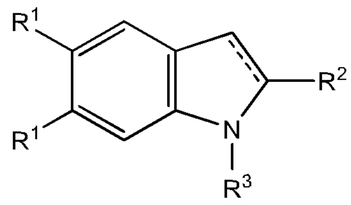

(1)

wherein the broken line represents the presence or absence of a $\pi$ bond; $R^1$ represents a hydroxy group or an acetoxy group; $R^2$ represents a hydrogen atom or -COOR,
wherein R represents a hydrogen atom, a methyl group, or an ethyl group; and $R^3$ represents a hydrogen atom, an acetyl group, a methyl group, or an ethyl group, and

(B2) an alkali agent.

[2] A method for improving color retention of hair dyed in the following step (I), comprising in order the following step (I) and step (II):

(I) applying a hair dye composition A comprising the following component (A1) and component (A2) to hair to dye the hair:

(A1) an oxidation dye, and
(A2) hydrogen peroxide; and

(II) applying a composition B comprising the following component (B1) and component (B2) and having pH of 8.0 or higher and 11.0 or lower to the hair:

(B1) a compound of the following formula (1) or a salt thereof:

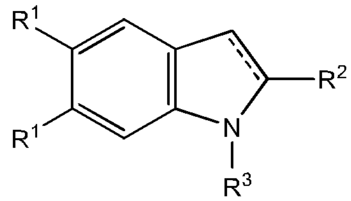

(1)

wherein the broken line represents the presence or absence of a $\pi$ bond; $R^1$ represents a hydroxy group or an acetoxy group; $R^2$ represents a hydrogen atom or -COOR,
wherein R represents a hydrogen atom, a methyl group, or an ethyl group; and $R^3$ represents a hydrogen atom, an acetyl group, a methyl group, or an ethyl group, and

(B2) an alkali agent.

[3] A method for improving color retention of artificially dyed hair, comprising the following step (IIa) :

(IIa) applying a composition B comprising the following component (B1) and component (B2) and having pH of 8.0 or higher and 11.0 or lower to the artificially dyed hair:

(B1) a compound of the following formula (1) or a salt thereof:

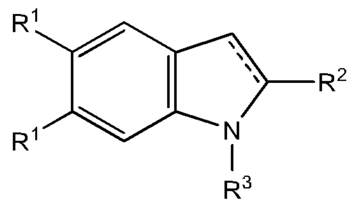

(1)

wherein the broken line represents the presence or absence of a $\pi$ bond; $R^1$ represents a hydroxy group or an acetoxy group; $R^2$ represents a hydrogen atom or -COOR,
wherein R represents a hydrogen atom, a methyl group, or an ethyl group; and $R^3$ represents a hydrogen atom, an acetyl group, a methyl group, or an ethyl group, and

(B2) an alkali agent.

[4] A kit for dyeing hair, comprising:

a hair dye composition A comprising the following component (A1) and component (A2):

(A1) an oxidation dye, and
(A2) hydrogen peroxide; and

a composition B comprising the following component (B1) and component (B2) and having pH of 8.0 or higher and 11.0 or lower:

(B1) a compound of the following formula (1) or a salt thereof:

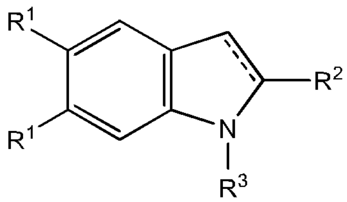

(1)

wherein the broken line represents the presence or absence of a $\pi$ bond; $R^1$ represents a hydroxy group or an acetoxy group; $R^2$ represents a hydrogen atom or -COOR,
wherein R represents a hydrogen atom, a methyl group, or an ethyl group; and $R^3$ represents a hydrogen atom, an acetyl group, a methyl group, or an ethyl group, and

(B2) an alkali agent.

Detailed Description of the Invention

However, the method disclosed in Patent Literature 1 might cause thermal damage on hair because heat treatment is essential.
The pretreatment agent composition for dyeing hair disclosed in Patent Literature 2 contains a direct dye and might therefore influence the color of hair after dyeing.
All the methods described in Patent Literatures 3 to 6 are treatment methods combined with a water-soluble base and thus easily cause color loss by hair wash after hair dyeing and have limitations to improvement in color retention.
The present invention relates to a hair treatment method which offers less discoloration of hair after dyeing and is capable of improving color retention.
The present inventors found that the above problems can be solved by a hair treatment method including in order a step (I) of applying a certain hair dye composition to hair to dye the hair, and a step (II) of applying a composition containing certain components to the hair.
The present invention can provide a hair treatment method which offers less discoloration of hair after dyeing and is capable of improving color retention.

[Definition]

In the present specification, the phrase "less discoloration of hair after dyeing" means that the color of hair subjected to a step (I) before being subjected to a step (II) has small difference from the color of hair subjected to the step (I) and the step (II). The discoloration of hair can be evaluated from change in hue angle h ($\Delta$h) and, specifically, can be evaluated by a method described in Examples.
The hue angle h is a value calculated according to the following equation from an a* value and a b* value defined by the CIE1976 L*a*b* color system.

$$\text{Hue angle } h = \tan^{-1}(b^* / a^*)$$

In the present specification, the phrase "improvement in color retention of hair after dyeing" means small color loss even if hair subjected to the step (I) and the step (II) is washed, and in particular, small difference in L* value (ΔL*) of hair between before and after hair wash. The L* value means a lightness L* defined by the CIE1976 L*a*b* color system. Specifically, the L* value and ΔL* of hair can be evaluated by methods described in Examples.

[Hair treatment method]

The hair treatment method of the present invention (hereinafter, also referred to as the "hair treatment method" or simply as the "treatment method of the present invention ") includes in order the following step (I) and step (II):

(I) applying the hair dye composition A containing the following component (A1) and component (A2) to hair to dye the hair:

(A1) an oxidation dye, and
(A2) hydrogen peroxide; and

(II) applying a composition B containing the following component (B1) and component (B2) and having pH of 8.0 or higher and 11.0 or lower to the hair:

(B1) a compound of the following formula (1) or a salt thereof:

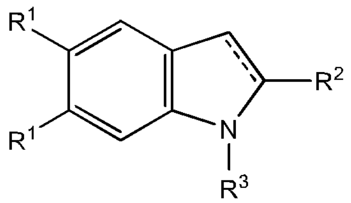

(1)

wherein the broken line represents the presence or absence of a π bond; $R^1$ represents a hydroxy group or an acetoxy group; $R^2$ represents a hydrogen atom or -COOR (wherein R represents a hydrogen atom, a methyl group, or an ethyl group); and $R^3$ represents a hydrogen atom, an acetyl group, a methyl group, or an ethyl group, and
(B2) an alkali agent.

The treatment method of the present invention, by having the configuration described above, can improve color retention of hair dyed in the step (I) while suppressing its discoloration.
The present invention exerts the effects described above, presumably because:
in the step (I), hair is dyed with the hair dye composition A containing (A1) an oxidation dye which is a coloring component, and (A2) hydrogen peroxide which is an oxidizing agent. The composition B for use in the step (II) to be subsequently performed contains a component (B1) which is a melanin precursor, and (B2) an alkali agent. When the composition B is applied to hair, the component (B1) penetrates the inside of the hair and is polymerized by air oxidation. Then, the component (B1) and its polymer remain in a cuticle layer positioned outside the cortex of the hair. This can presumably prevent the oxidation dye from dropping out of hair dyed in the step (I) and improve color retention.
The component (B2) in the composition B exerts an effect of promoting the oxidative polymerization within hair of the component (B1) by adjusting pH to the range of 8.0 or higher and 11.0 or lower. The component (B2) can presumably enhance an effect of preventing the oxidation dye from dropping out of hair and improve color retention through the action of swelling hair so as to open cuticle and allowing the component (B1) to efficiently penetrate the inside of the hair.
The component (B1) becomes a melanin-like substance by oxidative polymerization and is also capable of acting as a colorant and however, can presumably reduce the discoloration of hair as compared with a case of using other oxidation dyes or direct dyes.

<Step (I)>

The step (I) involves applying a hair dye composition A containing the following component (A1) and component (A2) to hair to dye the hair:

(A1) an oxidation dye, and
(A2) hydrogen peroxide

(Formulation)

A formulation of the hair dye composition A is not particularly limited and the hair dye composition A may be prepared in any formulation, for example, a liquid, a foam, a paste, a cream, a solid, or a powder. The formulation of the hair dye composition A is preferably a liquid, a foam, a paste, or a cream from the viewpoint of application to hair by coating, immersion, or the like.

The hair dye composition A may be a one-part composition or may be a multi-part composition such as a two-part composition. When the hair dye composition A is a multi-part composition, the phrase "content of a component X in the hair dye composition A" means a content of the component X in the hair dye composition A obtained by mixing multiple parts.

(Component (A1): oxidation dye)

Examples of the oxidation dye for use in the component (A1) include known oxidation dyes constituted by a precursor and a coupler.

Examples of the precursor in the oxidation dye include one or more members selected from the group consisting of paraphenylenediamine, toluene-2,5-diamine, 2-chloro-paraphenylenediamine, N-methoxyethylparaphenylenediamine, N,N-bis(2-hydroxyethyl)paraphenylenediamine, 2-(2-hydroxyethyl)paraphenylenediamine, 2,6-dimethylparaphenylenediamine, 4,4'-diaminodiphenylamine, 1,3-bis(N-(2-hydroxyethyl)-N-(4-aminophenyl)amino)-2-propanol, PEG-3,3,2'-paraphenylenediamine, paraaminophenol, paramethylaminophenol, 3-methyl-4-aminophenol [= 4-aminometacresol], 2-aminomethyl-4-aminophenol, 2-(2-hydroxyethylaminomethyl)-4-aminophenol, orthoaminophenol, 2-amino-5-methylphenol, 2-amino-6-methylphenol, 2-amino-5-acetamidophenol, 3,4-diaminobenzoic acid, 5-aminosalicylic acid, 2,4,5,6-tetraaminopyrimidine, 2,5,6-triamino-4-hydroxypyrimidine, 4,5-diamino-1-(4'-chlorobenzyl)pyrazole, 1-hydroxyethyl-4,5-diaminopyrazole, and a salt thereof.

Examples of the coupler in the oxidation dye include one or more members selected from the group consisting of metaphenylenediamine, 2,4-diaminophenoxyethanol, 2-amino-4-(2-hydroxyethylamino)anisole [= 2-amino-4-(β-hydroxyethyl)aminoanisole], 2,4-diamino-5-methylphenetole, 2,4-diamino-5-(2-hydroxyethoxy)toluene, 2,4-dimethoxy-1,3-diaminobenzene, 2,6-bis(2-hydroxyethylamino)toluene, 2,4-diamino-5-fluorotoluene, 1,3-bis(2,4-diaminophenoxy)propane, metaaminophenol, 2-methyl-5-aminophenol [= 5-aminoorthocresol], 2-methyl-5-(2-hydroxyethylamino)phenol, 2,4-dichloro-3-aminophenol, 2-chloro-3-amino-6-methylphenol, 2-methyl-4-chloro-5-aminophenol, N-cyclopentyl-metaaminophenol, 2-methyl-4-methoxy-5-(2-hydroxyethylamino)phenol, 2-methyl-4-fluoro-5-aminophenol, resorcinol, 2-methylresorcinol, 4-chlororesorcinol, 1-naphthol, 1,5-dihydroxynaphthalene, 1,7-dihydroxynaphthalene, 2,7-dihydroxynaphthalene, 2-isopropyl-5-methylphenol, 4-hydroxyindole, 5-hydroxyindole, 6-hydroxyindole, 7-hydroxyindole, 6-hydroxybenzomorpholine, 3,4-methylenedioxyphenol, 2-bromo-4,5-methylenedioxyphenol, 3,4-methylenedioxyaniline, 1-(2-hydroxyethyl)amino-3,4-methylenedioxybenzene, 2,6-dihydroxy-3,4-dimethylpyridine, 2,6-dimethoxy-3,5-diaminopyridine, 2,3-diamino-6-methoxypyridine, 2-methylamino-3-amino-6-methoxypyridine, 2-amino-3-hydroxypyridine, 2,6-diaminopyridine, and a salt thereof.

One or more of these oxidation dyes can be used as the component (A1) in accordance with a desired color. Only any one of the precursor and the coupler may be contained as the component (A1), or both the precursor and the coupler may be contained.

A content of the component (A1) in the hair dye composition A is preferably 0.005% by mass or more, more preferably 0.01% by mass or more, further more preferably 0.025% by mass or more, even more preferably 0.05% by mass or more, even more preferably 0.1% by mass or more, from the viewpoint of improvement in hair dyeability, and preferably 5.0% by mass or less, more preferably 2.0% by mass or less, from the viewpoint of reduction in discoloration of hair dyed in the step (I) and improvement in its color retention.

(Component (A2): hydrogen peroxide)

The component (A2) hydrogen peroxide acts as an oxidizing agent for the component (A1) and contributes to improvement in hair dyeability. The component (A2) is preferably blended in the state of hydrogen peroxide water into the hair dye composition A.

A content of the component (A2) in the hair dye composition A is preferably 0.1% by mass or more, more preferably 0.5% by mass or more, further more preferably 1.0% by mass or more, from the viewpoint of improvement in hair dyeability, and preferably 5.0% by mass or less, more preferably 4.0% by mass or less, from the viewpoint of the suppression of hair damage.

When the hair dye composition A is a multi-part composition, the component (A1) and the component (A2) are

preferably contained in separate preparations from the viewpoint of storage stability.

(Other components)

The hair dye composition A may appropriately contain, in addition to the components described above, components which are conventionally used in hair dye compositions, without impairing the object of the present invention. Examples of the components include pH adjusters, surfactants, viscosity adjusters, silicone, aromatic alcohols, coloring components other than the component (A1), polymers, oil agents, anti-dandruff agents, vitamin preparations, germicides, anti-inflammatory agents, antiseptics, chelating agents, humectants, pearlescent agents, a ceramide, fragrances, ultraviolet absorbers, and aqueous media.
In the present specification, the coloring component means a colorant or a precursor thereof. Examples of the coloring component other than the component (A1) include colorants or precursors thereof which are conventionally used in hair dye compositions except for the component (A1) and include the component (B1), direct dyes, and pigments.
A method for preparing the hair dye composition A is not particularly limited, and the hair dye composition A can be prepared by a routine method.
A commercially available hair dye composition may be used as the hair dye composition A. The hair dye composition A may not be limited as long as the hair dye composition contains the component (A1) and the component (A2). A hair dye composition is preferred in which when gray hair is dyed with the hair dye composition, an L* value of the gray hair after dyeing is 50 or less, from the viewpoint of effectively exerting the advantageous effects of the present invention. The L* value of the gray hair after dyeing is preferably 45 or less, and preferably 20 or more, more preferably 25 or more, further more preferably 30 or more.
The phrase "when gray hair is dyed with the hair dye composition A, an L* value of the gray hair after dyeing is 50 or less" means that, for example, in using a commercially available hair dye composition as the hair dye composition A, when gray hair is dyed with a bath ratio and a method designated for the hair dye composition, an L* value of the gray hair after dyeing is 50 or less.
In this context, the gray hair is preferably hair having an L* value of 65 or more and 75 or less. For example, "BM-W-A" manufactured by Beaulax Co., Ltd. can be used.
The step (I) involves applying the hair dye composition A to hair. When the hair dye composition A is a multi-part composition, the preparations are mixed before use to prepare the hair dye composition A, which is then applied to hair.
Examples of a method for applying the hair dye composition A to hair may not be limited as long as the method can contact the hair dye composition A with the hair. Examples thereof include a method of coating hair in a dry state or a wet state with the composition, and a method of immersing hair in a dry state or a wet state in the composition. Among those described above, a method of coating hair in a dry state with the hair dye composition is preferred from the viewpoint of improvement in hair dyeability.
An amount of the hair dye composition A applied to the hair is set to a bath ratio (dry mass of the hair : mass of the hair dye composition A) of preferably from 1 : 0.2 to 1 : 20, more preferably from 1 : 0.5 to 1 : 10, further more preferably from 1 : 0.5 to 1 : 5, even more preferably from 1 : 0.8 to 1 : 3, from the viewpoint of obtaining desired color tone.
The hair to which the hair dye composition A is to be applied is preferably head hair and can be at least a portion of the head hair.
After the step (I) of applying the hair dye composition A to hair, a step of leaving the resultant to stand is preferably further performed. In this respect, time for which the resultant is left to stand is preferably 1 minute or longer, more preferably 5 minutes or longer, further more preferably 10 minutes or longer, from the viewpoint of improvement in hair dyeability, and preferably 30 minutes or shorter, more preferably 25 minutes or shorter, from the viewpoint of reducing burden on a subject.

<Rinsing step>

The method of the present invention preferably includes, between the step (I) and the step (II), a step of washing off the hair dye composition A applied to the hair (hereinafter, also simply referred to as a "rinsing step").
The rinsing step is performed by washing off the hair dye composition A applied to the hair in the step (I) with water. Temperature of the water is not particularly limited, and warm water of 35 to 45°C is preferred.

<Drying step>

In the method of the present invention, after the rinsing step is performed subsequently to the step (I), the hair in a wet state may be subjected to the step (II) without a step of drying the hair. Alternatively, in the method of the present invention, after the rinsing step is performed subsequently to the step (I), a step of drying the hair (hereinafter, also simply referred to as a "drying step") may be performed to dry the hair so that the hair in a dry state is subjected to the step (II). The

step of drying the hair is a step of reducing an amount of water in the hair and includes, for example, performing one or more drying treatments selected from the group consisting of towel drying, blow (cold air or hot air) drying, and air drying in order to actively reduce the amount of water in the hair. Two or more of these drying treatments are preferably performed in combination.

In the method of the present invention, after the rinsing step is performed subsequently to the step (I), the hair in a wet state is preferably subjected to the step (II) without the step of drying the hair, from the viewpoint of shortening treatment time.

<Lightness (L* value) of hair>

An L* value of the hair dyed in the step (I) is preferably 50 or less, more preferably 45 or less, from the viewpoint of reduction in discoloration of the hair dyed in the step (I) and the efficacy of an effect of improving color retention. The L* value is preferably 20 or more, more preferably 25 or more, further more preferably 30 or more.

Specifically, the L* value of the hair can be measured by a method described in Examples.

In the present specification, the "hair dyed in the step (I)" means hair subjected to the step (I) before being subjected to the step (II) mentioned later. When the rinsing step is performed between the step (I) and the step (II), the "hair dyed in the step (I)" means hair subjected to the step (I) and the rinsing step before being subjected to the step (II).

<Hue angle (h value) of hair>

An h value of the hair dyed in the step (I) is preferably 5 or more, more preferably 10 or more, further more preferably 20 or more, even more preferably 30 or more, even more preferably 35 or more, and preferably 80 or less, more preferably 70 or less, further more preferably 60 or less, even more preferably 50 or less, from the viewpoint of reduction in discoloration of the hair dyed in the step (I) and the efficacy of an effect of improving color retention.

Specifically, the h value of the hair can be measured by a method described in Examples.

<Step (II)>

The step (II) involves applying a composition B containing the following component (B1) and component (B2) and having pH of 8.0 or higher and 11.0 or lower to the hair:

(B1) a compound of the following formula (1) or a salt thereof:

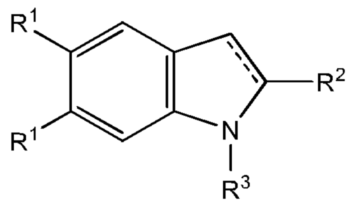

(1)

wherein the broken line represents the presence or absence of a π bond; $R^1$ represents a hydroxy group or an acetoxy group; $R^2$ represents a hydrogen atom or -COOR (wherein R represents a hydrogen atom, a methyl group, or an ethyl group); and $R^3$ represents a hydrogen atom, an acetyl group, a methyl group, or an ethyl group, and

(B2) an alkali agent.

(Formulation)

A formulation of the composition B is not particularly limited and may be any formulation, for example, a liquid, a foam, a paste, a cream, a solid, or a powder. The formulation of the composition B is preferably a liquid, a foam, a paste, or a cream from the viewpoint of application to hair by coating, immersion, or the like.

The composition B may be a one-part composition or may be a multi-part composition such as a two-part composition, and is preferably a one-part composition.

(Component (B1): compound of formula (1) or salt thereof)

The composition B contains a component (B1) which is a compound of the formula (1) or a salt thereof. The component (B1) is a melanin precursor which is polymerized by air oxidation and thereby converted to a melanin pigment,

and acts as a color retention improver for the hair dyed in the step (I) in the present invention.

The compound of the formula (1) is an indole derivative or an indoline derivative, or a salt thereof. In the present invention, one or more thereof can be used (in combination).

The component (B1) is more preferably an indole derivative (i.e., a $\pi$ bond is present in a moiety of the broken line in the formula (1)) from the viewpoint of reduction in discoloration of hair dyed in the step (I) and improvement in its color retention.

In the formula (1), $R^1$ is preferably a hydroxy group, and $R^2$ is preferably a hydrogen atom or -COOR (wherein R is a hydrogen atom, a methyl group, or an ethyl group), more preferably a hydrogen atom or -COOH, from the viewpoint of availability of the component (B1), reduction in discoloration of hair dyed in the step (I), and improvement in its color retention. $R^3$ is preferably a hydrogen atom.

Examples of the compound of the formula (1) include 5,6-dihydroxyindole, 5,6-dihydroxyindole-2-carboxylic acid, methyl 5,6-dihydroxyindole-2-carboxylate, ethyl 5,6-dihydroxyindole-2-carboxylate, N-methyl-5,6-dihydroxyindole, N-methyl-5,6-dihydroxyindole-2-carboxylic acid, N-ethyl-5,6-dihydroxyindole, N-ethyl-5,6-dihydroxyindole-2-carboxylic acid, N-acetyl-5,6-dihydroxyindole, N-acetyl-5,6-dihydroxyindole-2-carboxylic acid, 5-acetoxy-6-hydroxyindole, 5-acetoxy-6-hydroxyindole-2-carboxylic acid, 5,6-dihydroxyindoline, 5,6-dihydroxyindoline-2-carboxylic acid, methyl 5,6-dihydroxyindoline-2-carboxylate, ethyl 5,6-dihydroxyindoline-2-carboxylate, N-methyl-5,6-dihydroxyindoline, N-methyl-5,6-dihydroxyindoline-2-carboxylic acid, N-ethyl-5,6-dihydroxyindoline, N-ethyl-5,6-dihydroxyindoline-2-carboxylic acid, N-acetyl-5,6-dihydroxyindoline, N-acetyl-5,6-dihydroxyindoline-2-carboxylic acid, 5-acetoxy-6-hydroxyindoline, and 5-acetoxy-6-hydroxyindoline-2-carboxylic acid, one or more of which can be used.

Examples of the salt of the compound of the formula (1) include hydrochloride, hydrobromide, sulfate, phosphate, acetate, propionate, lactate, and citrate of any of the compounds. Hydrobromide is preferred from the viewpoint of availability, reduction in discoloration of hair dyed in the step (I), and improvement in its color retention.

When $R^2$ in the formula (1) is -COOH, examples of the salt of the compound of the formula (1) include carboxylate thereof (wherein $R^2$ is $-COO^-X^+$ ($X^+$ is a cation such as an alkali metal ion (e.g., $Na^+$ and $K^+$), an alkaline earth metal ion (e.g., $Ca^+$ and $Mg^+$), or an ammonium ion)).

The component (B1) is preferably one or more members selected from the group consisting of 5,6-dihydroxyindole, 5,6-dihydroxyindole-2-carboxylic acid, 5,6-dihydroxyindoline, 5,6-dihydroxyindoline-2-carboxylic acid, and a salt thereof, is more preferably one or more members selected from the group consisting of 5,6-dihydroxyindole, 5,6-dihydroxyindole-2-carboxylic acid, and 5,6-dihydroxyindoline hydrobromide, is further more preferably one or more members selected from the group consisting of 5,6-dihydroxyindole and 5,6-dihydroxyindole-2-carboxylic acid, and even more preferably contains 5,6-dihydroxyindole, from the viewpoint of reduction in discoloration of hair dyed in the step (I) and improvement in its color retention.

When the component (a1) contains 5,6-dihydroxyindole and 5,6-dihydroxyindole-2-carboxylic acid, a molar ratio between the 5,6-dihydroxyindole and the 5,6-dihydroxyindole-2-carboxylic acid is preferably in the range of from 50 : 50 to 99 : 1, more preferably in the range of from 80 : 20 to 99 : 1, further more preferably in the range of from 85 : 15 to 95 : 5, from the viewpoint of improvement in its color retention.

When the component (a1) contains 5,6-dihydroxyindole and 5,6-dihydroxyindole-2-carboxylic acid, the molar ratio between the 5,6-dihydroxyindole and the 5,6-dihydroxyindole-2-carboxylic acid in the component (a1) can be quantified by reverse-phase HPLC.

A content of the component (B1) in the composition B is preferably 0.01% by mass or more, more preferably 0.02% by mass or more, further more preferably 0.03% by mass or more, even more preferably 0.05% by mass or more, from the viewpoint of improvement in color retention, and preferably 3.0% by mass or less, more preferably 2.0% by mass or less, further more preferably 1.0% by mass or less, even more preferably 0.8% by mass or less, even more preferably 0.5% by mass or less, even more preferably 0.4% by mass or less, even more preferably 0.3% by mass or less, even more preferably 0.25% by mass or less, from the viewpoint of reduction in discoloration of hair dyed in the step (I).

(Component (B2): alkali agent)

The component (B2) exerts an effect of improving color retention of hair dyed in the step (I) through the effect of swelling hair so as to open cuticle and allowing the component (B1) to penetrate the inside of the hair.

Any alkali agent for use in a usual hair dye composition can be used without particular limitations as the component (B2), which may be any of inorganic alkali agents and organic alkali agents. The component (B2) is preferably ammonia or an organic alkali agent from the viewpoint of improvement in its color retention of hair dyed in the step (I).

Examples of the alkali agent which is preferred as the component (B2) include: ammonia; alkanolamine such as mono-, di-, or trimethanolamine, mono-, di-, or triethanolamine, trishydroxymethylaminomethane, and 2-amino-2-methyl-1-propanol; alkylamine such as methylamine, dimethylamine, ethylamine, diethylamine, N-methylethylamine, propylamine, and butylamine; and aralkylamine such as benzylamine, one or more of which can be used. The alkanolamine, the alkylamine, or the aralkylamine has preferably 1 or more and 10 or less, more preferably 1 or more

and 8 or less carbon atoms from the viewpoint of water solubility.

Among those described above, the component (B2) is preferably one or more members selected from the group consisting of ammonia, alkanolamine, alkylamine, and aralkylamine, more preferably contains one or more members selected from the group consisting of ammonia and alkanolamine, and further more preferably contains alkanolamine, from the viewpoint of improvement in its color retention of hair dyed in the step (I). The component (B2) preferably contains one or more members selected from the group consisting of monoethanolamine, trishydroxymethylaminomethane, and 2-amino-2-methyl-1-propanol, more preferably contains one or more members selected from the group consisting of monoethanolamine and 2-amino-2-methyl-1-propanol, and further more preferably contains monoethanolamine, as the alkanolamine from the viewpoint of improvement in its color retention of hair dyed in the step (I).

A content of the alkanolamine in the component (B2) is preferably 70% by mass or more, more preferably 80% by mass or more, further more preferably 90% by mass or more, and 100% by mass or less, from the viewpoint of improvement in its color retention of hair dyed in the step (I).

A content of the component (B2) in the composition B is preferably 0.01% by mass or more, more preferably 0.1% by mass or more, further more preferably 0.5% by mass or more, even more preferably 1.0% by mass or more, even more preferably 2.0% by mass or more, from the viewpoint of improvement in its color retention of hair dyed in the step (I), and preferably 10% by mass or less, more preferably 7.5% by mass or less, further more preferably 5.0% by mass or less, from the viewpoint of suppressing hair damage.

[Other components]

The composition B may appropriately contain, in addition to the components described above, components which are conventionally used in hair dye compositions, without impairing the object of the present invention. Examples of the components include antioxidants, pH adjusters, surfactants, viscosity adjusters, silicone, aromatic alcohols, aqueous media such as water, lower alcohols, low-molecular diol, and triol, polymers, oil agents, anti-dandruff agents, vitamin preparations, germicides, anti-inflammatory agents, antiseptics, chelating agents, humectants, pearlescent agents, a ceramide, fragrances, and ultraviolet absorbers. Among them, the composition B preferably contains water and further contains one or more members selected from the group consisting of an antioxidant, a pH adjuster, and a surfactant.

[Antioxidant]

The composition B can contain an antioxidant from the viewpoint of suppressing the oxidative polymerization of the component (B1) outside hair.

Examples of the antioxidant include sulfurous acid, pyrosulfurous acid, hydrogen sulfite, sulfur dioxide, L-ascorbic acid, thioglycolic acid, L-cysteine, N-acetyl-L-cysteine, and a salt thereof, and a derivative thereof. Examples of the salt include alkali metals such as sodium and potassium.

Among those described above, the antioxidant is preferably one or more members selected from the group consisting of sulfite, pyrosulfite, hydrosulfite, sulfur dioxide, L-ascorbic acid, and L-ascorbate, more preferably one or more members selected from the group consisting of sodium sulfite and sodium L-ascorbate, further more preferably sodium sulfite, from the viewpoint of the effect of suppressing the oxidative polymerization of the component (B1) outside hair.

When the composition B contains an antioxidant, a content of the antioxidant in the composition B is preferably 0.01% by mass or more, more preferably 0.05% by mass or more, further more preferably 0.1% by mass or more, even more preferably 0.2% by mass or more, and preferably 10% by mass or less, more preferably 5.0% by mass or less, further more preferably 3.0% by mass or less, even more preferably 2.0% by mass or less, even more preferably 1.0% by mass or less, from the viewpoint of suppressing the oxidative polymerization of the component (B1) outside hair.

[pH adjuster]

The composition B can contain a pH adjuster from the viewpoint of adjusting pH to a desired range mentioned later and accelerating the polymerization reaction of the component (B1) within hair.

Since the composition B contains the alkali agent as the component (B2), the pH adjuster for use in the composition B is preferably a protonating agent. The protonating agent may be any of monobasic acids and polybasic acids or may be any of organic acids (having 1 or more and 8 or less carbon atoms, except for ascorbic acid) and inorganic acids.

Examples of the protonating agent include one or more members selected from the group consisting of hydrochloric acid, sulfuric acid, phosphoric acid, formic acid, acetic acid, and citric acid. One or more members selected from the group consisting of phosphoric acid and citric acid is preferred from the viewpoint of ease of adjusting pH of the composition B to a range mentioned later.

When the composition B contains the pH adjuster, a content of the pH adjuster in the composition B is preferably

0.05% by mass or more, more preferably 0.1% by mass or more, further more preferably 0.2% by mass or more, and preferably 5.0% by mass or less, more preferably 4.0% by mass or less, further more preferably 3.5% by mass or less, from the viewpoint of ease of adjusting pH of the composition B to a range mentioned later.

[Surfactant]

The composition B can contain a surfactant for the purpose of, for example, suppressing hair damage and improving feel.

Examples of the surfactant include cationic surfactants, anionic surfactants, amphoteric surfactants, and nonionic surfactants, one or more of which can be used. When the composition B is a conditioning agent or a treatment agent, the composition B preferably contains at least a cationic surfactant as the surfactant.

When the composition B contains a surfactant, a content of the surfactant in the composition B is preferably 0.1% by mass or more, more preferably 0.5% by mass or more, further more preferably 1.0% by mass or more, and preferably 30% by mass or less, more preferably 20% by mass or less, from the viewpoint of the suppression of hair damage, improvement in feel, and the like.

The composition B preferably substantially contains no coloring component from the viewpoint of reducing discoloration of hair dyed in the step (I). The phrase "substantially contain no coloring component" means that a content of the coloring component in the composition B is preferably 1% by mass or less, more preferably 0.5% by mass or less, further more preferably 0.2% by mass or less, even more preferably 0% by mass.

In this context, examples of the coloring component include the oxidation dye serving as the component (A1), direct dyes, and pigments.

Preferably, the composition B substantially contains no oxidizing agent from the viewpoint of suppressing hair damage. The phrase "composition B substantially contains no oxidizing agent" means that a content of the oxidizing agent in the composition B is preferably less than 0.1% by mass, even more preferably less than 0.01% by mass, further more preferably 0% by mass.

Examples of the oxidizing agent include peroxide such as hydrogen peroxide, urea peroxide, and melamine peroxide. Hydrogen peroxide is preferred. The content of the oxidizing agent means the content of an active ingredient in the oxidizing agent. In using, for example, hydrogen peroxide water, as the oxidizing agent, the content of the oxidizing agent means the content of hydrogen peroxide.

A method for preparing the composition B is not particularly limited. This part can be prepared, for example, by blending the component (B1), the component (B2), an aqueous medium, and other optional components and mixing these components by using a known stirring apparatus or the like. The composition B is preferably prepared in an inert gas (e.g., nitrogen gas) atmosphere from the viewpoint of avoiding polymerization of the component (B1).

(pH)

The pH of the composition B is preferably 8.0 or higher, more preferably 9.0 or higher, further more preferably 9.5 or higher, even more preferably 9.8 or higher, from the viewpoint of improvement in its color retention of hair dyed in the step (I). The pH is preferably 11.0 or lower, more preferably 10.8 or lower, further more preferably 10.5 or lower, from the viewpoint of suppressing hair damage.

The pH is a value measured at 25°C and, specifically, can be measured in accordance with a method described in Examples.

Examples of a method for applying the composition B to the hair in the step (II) may not be limited as long as the method facilitates contacting the composition B with the hair. Examples thereof include a method of coating hair in a dry state or a wet state with the composition B, and a method of immersing hair in a dry state or a wet state in the composition. Among those described above, a method of coating hair in a wet state with the composition B is preferred.

An amount of the composition B applied to the hair is a bath ratio (mass of the hair : mass of the composition B) of preferably from 1 : 0.1 to 1 : 20, more preferably from 1 : 0.2 to 1 : 10, further more preferably from 1 : 0.3 to 1 : 5, even more preferably from 1 : 0.4 to 1 : 3, from the viewpoint of reduction in discoloration of hair dyed in the step (I) and improvement in its color retention. In this context, the "mass of the hair" means the mass of the hair in a dry state and refers to the mass of the hair in a dry state even if the hair is in a wet state.

After the step (II) of applying the composition B to the hair, a step of leaving the resultant to stand is preferably further performed. In this respect, time for which the resultant is left to stand is preferably 1 minute or longer, more preferably 3 minutes or longer, from the viewpoint of improvement in color retention, and preferably 30 minutes or shorter, more preferably 20 minutes or shorter, from the viewpoint of reducing burden on a subject.

<Rinsing step>

The method of the present invention preferably contains, after the step (II), the step of rinsing the composition B applied to the hair (hereinafter, also simply referred to as a "rinsing step"). The rinsing step is performed, for example, by washing off the composition B applied to the hair with water. Temperature of the water is not particularly limited, and warm water of 35 to 45°C is preferred.

<Drying step>

The method of the present invention preferably includes, after the rinsing step, the step of drying the hair (drying step). The step of drying the hair includes performing at least one or more drying treatments selected from the group consisting of towel drying, blow (cold air or hot air) drying, and air drying. Two or more of these drying treatments are preferably performed in combination.

[Method for improving color retention of hair]

The present invention provides a method for improving color retention of hair dyed in the following step (I), including in order the following step (I) and step (II):

(I) applying a hair dye composition A containing the following component (A1) and component (A2) to hair to dye the hair:

(A1) an oxidation dye, and
(A2) hydrogen peroxide; and

(II) applying a composition B containing the following component (B1) and component (B2) and having pH of 8.0 or higher and 11.0 or lower to the hair:

(B1) a compound of the following formula (1) or a salt thereof:

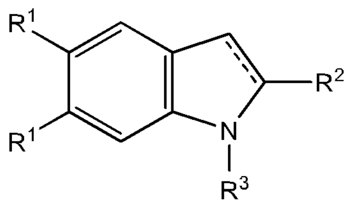

(1)

wherein the broken line represents the presence or absence of a π bond; $R^1$ represents a hydroxy group or an acetoxy group; $R^2$ represents a hydrogen atom or -COOR (wherein R represents a hydrogen atom, a methyl group, or an ethyl group); and $R^3$ represents a hydrogen atom, an acetyl group, a methyl group, or an ethyl group, and
(B2) an alkali agent.

The step (I), the step (II), and their preferred forms are the same as those described above.

The present invention further provides a method for improving color retention of artificially dyed hair, including the following step (IIa):
(IIa) applying a composition B containing the following component (B1) and component (B2) and having pH of 8.0 or higher and 11.0 or lower to the artificially dyed hair:

(B1) a compound of the following formula (1) or a salt thereof:

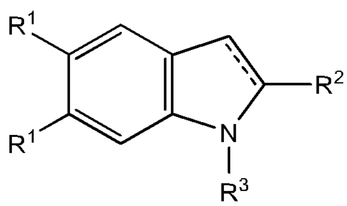

(1)

wherein the broken line represents the presence or absence of a π bond; $R^1$ represents a hydroxy group or an acetoxy group; $R^2$ represents a hydrogen atom or -COOR (wherein R represents a hydrogen atom, a methyl group, or an ethyl group); and $R^3$ represents a hydrogen atom, an acetyl group, a methyl group, or an ethyl group, and
(B2) an alkali agent.

The "artificially dyed hair" means hair treated by dyeing with a colorant such as a pigment or a dye. The colorant is preferably one or more members selected from the group consisting of a pigment, a direct dye, and an oxidation dye from the viewpoint of improvement in color retention, more preferably one or more members selected from the group consisting of a direct dye and an oxidation dye from the viewpoint of the efficacy of an effect of improving color retention, further more preferably an oxidation dye.

The pigment may not be limited as long as the pigment is conventionally used in hair dye composition and the like. Examples thereof include: white inorganic pigments such as titanium oxide, zinc oxide, cerium oxide, and barium sulfate; color inorganic pigments such as yellow oxide of iron, black oxide of iron, red iron oxide, carbon black, chromium oxide, chromium hydroxide, iron blue, and ultramarine; photoluminescent powders such as titanium oxide-coated mica, titanium oxide-coated bismuth oxychloride, iron oxide-coated titanated mica, iron oxide mica, iron blue-treated titanated mica, carmine-treated titanated mica, bismuth oxychloride, and argentine; organic pigments such as Red No. 201, Red No. 202, Red No. 205, Red No. 226, Red No. 228, Orange No. 203, Orange No. 204, Blue No. 404, and Yellow No. 401; lake pigments including zirconium, barium, or aluminum lake, such as Red No. 3, Red No. 104, Red No. 106, Orange No. 205, Yellow No. 4, Yellow No. 5, Green No. 3, and Blue No. 1; and composite pigments such as fine particles of titanium oxide-coated titanated mica, fine particles of zinc oxide-coated titanated mica, barium sulfate-coated titanated mica, titanium oxide-containing silicon dioxide, and zinc oxide-containing silicon dioxide, one or more of which can be used.

Each of these pigments may be surface-treated with various surface treatment agents for use. The surface treatment is not particularly limited, and various surface treatments, for example, fluorine compound treatment, silicone treatment, silicone resin treatment, pendant treatment, silane coupling agent treatment, titanium coupling agent treatment, oil agent treatment, metallic soap treatment, N-acylated lysine treatment, polyethylene glycol treatment, polyvinyl alcohol (PVA) treatment, polyacrylic acid treatment, hyaluronic acid treatment, alginic acid treatment, inorganic compound treatment, plasma treatment, or mechanochemical treatment can be performed beforehand.

Examples of the direct dye include acid dyes, nitro dyes, disperse dyes, basic dyes, and direct dyes described in JP-A-2003-342139.

Examples of the acid dye include Blue No. 1, Violet No. 401, Black No. 401, Orange No. 205, Red No. 227, Red No. 106, Yellow No. 203, and Acid Orange 3. Examples of the nitro dye include 2-nitroparaphenylenediamine, 2-amino-6-chloro-4-nitrophenol, 3-nitro-p-hydroxyethylaminophenol, 4-nitroorthophenylenediamine, 4-amino-3-nitrophenol, 4-hydroxypropylamino-3-nitrophenol, HC Blue 2, HC Orange 1, HC Red 1, HC Yellow 2, HC Yellow 4, HC Yellow 5, HC Red 3, and N,N-bis-(2-hydroxyethyl)-2-nitroparaphenylenediamine. Examples of the disperse dye include Disperse Violet 1, Disperse Blue 1, and Disperse Black 9. Examples of the basic dye include Basic Blue 99, Basic Brown 16, Basic Brown 17, Basic Red 76, Basic Red 51, Basic Yellow 57, Basic Yellow 87, and Basic Orange 31. One or more of these direct dyes can be used.

Examples of the oxidation dye include the components listed in the component (A1).

The artificially dyed hair can be, for example, hair dyed in accordance with a routine method by using a commercially available hair dye composition.

An L* value of the artificially dyed hair to be subjected to the step (IIa) is preferably 50 or less, more preferably 45 or less, from the viewpoint of the efficacy of an effect of improving color retention. The L* value is preferably 20 or more, more preferably 25 or more, further more preferably 30 or more.

An h value of the artificially dyed hair to be subjected to the step (IIa) is preferably 5 or more, more preferably 10 or more, further more preferably 20 or more, even more preferably 30 or more, even more preferably 35 or more, and preferably 80 or less, more preferably 70 or less, further more preferably 60 or less, even more preferably 50 or less, from the viewpoint of the efficacy of an effect of improving color retention.

The L* value and the h value of the hair can be measured by the same methods as those described above.

The step (IIa) and its preferred form are the same as those in the step (II) except that the hair to which the composition B is to be applied is the artificially dyed hair.

[Kit for dyeing hair]

The present invention also provides a kit for dyeing hair, containing:

a hair dye composition A containing the following component (A1) and component (A2):

(A1) an oxidation dye, and

(A2) hydrogen peroxide; and

a composition B containing the following component (B1) and component (B2) and having pH of 8.0 or higher and 11.0 or lower:

(B1) a compound of the following formula (1) or a salt thereof:

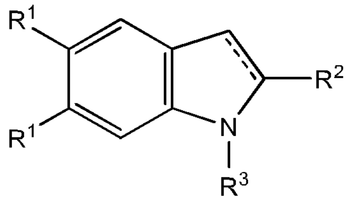

(1)

wherein the broken line represents the presence or absence of a $\pi$ bond; $R^1$ represents a hydroxy group or an acetoxy group; $R^2$ represents a hydrogen atom or -COOR (wherein R represents a hydrogen atom, a methyl group, or an ethyl group); and $R^3$ represents a hydrogen atom, an acetyl group, a methyl group, or an ethyl group, and
(B2) an alkali agent.

The hair dye composition A, the composition B, and their preferred forms are the same as those described above.
The kit for dyeing hair may not be limited as long as the kit contains the hair dye composition A and the composition B. Examples thereof include two-part kits in which the hair dye composition A and the composition B are individually enclosed in their respective containers. The shapes of the containers are not particularly limited and can be appropriately selected in accordance with formulations. Examples thereof include bottles, tubes, dispensers, and aerosols.
The kit for dyeing hair may further contain a preparation other than the hair dye composition A and the composition B.
The kit for dyeing hair may further have, if necessary, a comb, a brush, or the like for coating hair with the hair dye composition A and the composition B, for example.
The kit for dyeing hair of the present invention can be used, preferably, in accordance with the method described in the hair treatment method mentioned above.
Hereinafter, in relation to the embodiments mentioned above, the present invention further discloses the following.

<1> A hair treatment method comprising in order the following step (I) and step (II):

(I) applying the hair dye composition A comprising the following component (A1) and component (A2) to hair to dye the hair:

(A1) an oxidation dye, and
(A2) hydrogen peroxide; and

(II) applying a composition B comprising the following component (B1) and component (B2) and having pH of 8.0 or higher and 11.0 or lower to the hair:

(B1) a compound of the following formula (1) or a salt thereof:

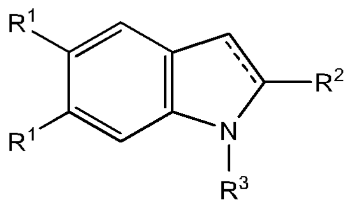

(1)

wherein the broken line represents the presence or absence of a $\pi$ bond; $R^1$ represents a hydroxy group or an acetoxy group; $R^2$ represents a hydrogen atom or -COOR (wherein R represents a hydrogen atom, a methyl

group, or an ethyl group); and $R^3$ represents a hydrogen atom, an acetyl group, a methyl group, or an ethyl group, and
(B2) an alkali agent.

<2> The hair treatment method according to <1>, wherein the hair dye composition A is in a liquid, a foam, a paste, a cream, a solid, or a powder, preferably a liquid, a foam, a paste, or a cream.
<3> The hair treatment method according to <1> or <2>, wherein the component (A1) is one or more members selected from the group consisting of oxidation dyes constituted by a precursor and a coupler.
<4> The hair treatment method according to <3>, wherein the precursor is one or more members selected from the group consisting of paraphenylenediamine, toluene-2,5-diamine, 2-chloro-paraphenylenediamine, N-methoxyethylparaphenylenediamine, N,N-bis(2-hydroxyethyl)paraphenylenediamine, 2-(2-hydroxyethyl)paraphenylenediamine, 2,6-dimethylparaphenylenediamine, 4,4'-diaminodiphenylamine, 1,3-bis(N-(2-hydroxyethyl)-N-(4-aminophenyl) amino)-2-propanol, PEG-3,3,2'-paraphenylenediamine, paraaminophenol, paramethylaminophenol, 3-methyl-4-aminophenol [= 4-aminometacresol], 2-aminomethyl-4-aminophenol, 2-(2-hydroxyethylaminomethyl)-4-aminophenol, orthoaminophenol, 2-amino-5-methylphenol, 2-amino-6-methylphenol, 2-amino-5-acetamidophenol, 3,4-diaminobenzoic acid, 5-aminosalicylic acid, 2,4,5,6-tetraaminopyrimidine, 2,5,6-triamino-4-hydroxypyrimidine, 4,5-diamino-1-(4'-chlorobenzyl)pyrazole, 1-hydroxyethyl-4,5-diaminopyrazole, and a salt thereof.
<5> The hair treatment method according to <3> or <4>, wherein the coupler is one or more members selected from the group consisting of metaphenylenediamine, 2,4-diaminophenoxyethanol, 2-amino-4-(2-hydroxyethylamino)anisole [= 2-amino-4-(β-hydroxyethyl)aminoanisole], 2,4-diamino-5-methylphenetole, 2,4-diamino-5-(2-hydroxyethoxy)toluene, 2,4-dimethoxy-1,3-diaminobenzene, 2,6-bis(2-hydroxyethylamino)toluene, 2,4-diamino-5-fluorotoluene, 1,3-bis(2,4-diaminophenoxy)propane, metaaminophenol, 2-methyl-5-aminophenol [= 5-aminoorthocresol], 2-methyl-5-(2-hydroxyethylamino)phenol, 2,4-dichloro-3-aminophenol, 2-chloro-3-amino-6-methylphenol, 2-methyl-4-chloro-5-aminophenol, N-cyclopentyl-metaaminophenol, 2-methyl-4-methoxy-5-(2-hydroxyethylamino) phenol, 2-methyl-4-fluoro-5-aminophenol, resorcinol, 2-methylresorcinol, 4-chlororesorcinol, 1-naphthol, 1,5-dihydroxynaphthalene, 1,7-dihydroxynaphthalene, 2,7-dihydroxynaphthalene, 2-isopropyl-5-methylphenol, 4-hydroxyindole, 5-hydroxyindole, 6-hydroxyindole, 7-hydroxyindole, 6-hydroxybenzomorpholine, 3,4-methylenedioxyphenol, 2-bromo-4,5-methylenedioxyphenol, 3,4-methylenedioxyaniline, 1-(2-hydroxyethyl)amino-3,4-methylenedioxybenzene, 2,6-dihydroxy-3,4-dimethylpyridine, 2,6-dimethoxy-3,5-diaminopyridine, 2,3-diamino-6-methoxypyridine, 2-methylamino-3-amino-6-methoxypyridine, 2-amino-3-hydroxypyridine, 2,6-diaminopyridine, and a salt thereof.
<6> The hair treatment method according to any one of <1> to <5>, wherein a content of the component (A1) in the hair dye composition A is preferably 0.005% by mass or more, more preferably 0.01% by mass or more, further more preferably 0.025% by mass or more, even more preferably 0.05% by mass or more, even more preferably 0.1% by mass or more, and preferably 5.0% by mass or less, more preferably 2.0% by mass or less.
<7> The hair treatment method according to any one of <1> to <6>, wherein a content of the component (A2) in the hair dye composition A is preferably 0.1% by mass or more, more preferably 0.5% by mass or more, further more preferably 1.0% by mass or more, and preferably 5.0% by mass or less, more preferably 4.0% by mass or less.
<8> The hair treatment method according to any one of <1> to <7>, wherein the hair dye composition A is a multi-part composition, and the component (A1) and the component (A2) are contained in separate preparations.
<9> The hair treatment method according to any one of <1> to <8>, wherein a method for applying the hair dye composition A to hair is a method of contacting the hair dye composition A with the hair, preferably a method of coating hair in a dry state or a wet state with the hair dye composition A or a method of immersing hair in a dry state or a wet state in the hair dye composition **A.**
<10> The hair treatment method according to any one of <1> to <9>, wherein an amount of the hair dye composition A applied to the hair is a bath ratio (dry mass of the hair : mass of the hair dye composition A) of preferably from 1 : 0.2 to 1 : 20, more preferably from 1 : 0.5 to 1 : 10, further more preferably from 1 : 0.5 to 1 : 5, even more preferably from 1 : 0.8 to 1 : 3.
<11> The hair treatment method according to any one of <1> to <10>, further comprising, after the step (I) of applying the hair dye composition A to hair, the step of leaving the resultant to stand, wherein time for which the resultant is left to stand is preferably 1 minute or longer, more preferably 5 minutes or longer, further more preferably 10 minutes or longer, and preferably 30 minutes or shorter, more preferably 25 minutes or shorter.
<12> The hair treatment method according to any one of <1> to <11>, further comprising, between the step (I) and the step (II), a rinsing step of washing off the hair dye composition A applied to the hair.
<13> The hair treatment method according to <12>, further comprising, after the rinsing step, a drying step of drying the hair, wherein a method of subjecting the hair in a dry state to the step (II), preferably one or more drying treatments selected from the group consisting of towel drying, blow (cold air or hot air) drying, and air drying is performed, and more preferably two or more of the drying treatments are performed in combination.

<14> The hair treatment method according to any one of <1> to <13>, wherein an L* value of the hair dyed in the step (I) is 50 or less, preferably 45 or less, and preferably 20 or more, more preferably 25 or more, further more preferably 30 or more.

<15> The hair treatment method according to any one of <1> to <14>, wherein a lightness (L* value) of the hair before dyeing to be subjected to the step (I) is preferably 65 or more and 75 or less.

<16> The hair treatment method according to any one of <1> to <15>, wherein a hue angle (h value) of the hair dyed in the step (I) is preferably 5 or more, more preferably 10 or more, further more preferably 20 or more, even more preferably 30 or more, even more preferably 35 or more, and preferably 80 or less, more preferably 70 or less, further more preferably 60 or less, even more preferably 50 or less.

<17> The hair treatment method according to any one of <1> to <16>, wherein the composition B is in a liquid, a foam, a paste, a cream, a solid, or a powder, preferably a liquid, a foam, a paste, or a cream.

<18> The hair treatment method according to any one of <1> to <17>, wherein the component (B1) is one or more members selected from the group consisting of 5,6-dihydroxyindole, 5,6-dihydroxyindole-2-carboxylic acid, methyl 5,6-dihydroxyindole-2-carboxylate, ethyl 5,6-dihydroxyindole-2-carboxylate, N-methyl-5,6-dihydroxyindole, N-methyl-5,6-dihydroxyindole-2-carboxylic acid, N-ethyl-5,6-dihydroxyindole, N-ethyl-5,6-dihydroxyindole-2-carboxylic acid, N-acetyl-5,6-dihydroxyindole, N-acetyl-5,6-dihydroxyindole-2-carboxylic acid, 5-acetoxy-6-hydroxyindole, 5-acetoxy-6-hydroxyindole-2-carboxylic acid, 5,6-dihydroxyindoline, 5,6-dihydroxyindoline-2-carboxylic acid, methyl 5,6-dihydroxyindoline-2-carboxylate, ethyl 5,6-dihydroxyindoline-2-carboxylate, N-methyl-5,6-dihydroxyindoline, N-methyl-5,6-dihydroxyindoline-2-carboxylic acid, N-ethyl-5,6-dihydroxyindoline, N-ethyl-5,6-dihydroxyindoline-2-carboxylic acid, N-acetyl-5,6-dihydroxyindoline, N-acetyl-5,6-dihydroxyindoline-2-carboxylic acid, 5-acetoxy-6-hydroxyindoline, and 5-acetoxy-6-hydroxyindoline-2-carboxylic acid, and hydrochloride, hydrobromide, sulfate, phosphate, acetate, propionate, lactate, and citrate of each of these compounds, preferably hydrobromide of each of these compounds.

<19> The hair treatment method according to any one of <1> to <18>, wherein the component (B1) is preferably one or more members selected from the group consisting of 5,6-dihydroxyindole, 5,6-dihydroxyindole-2-carboxylic acid, 5,6-dihydroxyindoline, 5,6-dihydroxyindoline-2-carboxylic acid, and a salt thereof, more preferably one or more members selected from the group consisting of 5,6-dihydroxyindole, 5,6-dihydroxyindole-2-carboxylic acid, and 5,6-dihydroxyindoline hydrobromide, further more preferably one or more members selected from the group consisting of 5,6-dihydroxyindole and 5,6-dihydroxyindole-2-carboxylic acid, and even more preferably the component (B1) comprises 5,6-dihydroxyindole.

<20> The hair treatment method according to any one of <1> to <19>, wherein a content of the component (B1) in the composition B is preferably 0.01% by mass or more, more preferably 0.02% by mass or more, further more preferably 0.03% by mass or more, even more preferably 0.05% by mass or more, and preferably 3.0% by mass or less, more preferably 2.0% by mass or less, further more preferably 1.0% by mass or less, even more preferably 0.8% by mass or less, even more preferably 0.5% by mass or less, even more preferably 0.4% by mass or less, even more preferably 0.3% by mass or less, even more preferably 0.25% by mass or less.

<21> The hair treatment method according to any one of <1> to <20>, wherein the component (B2) is an inorganic alkali agent or an organic alkali agent, preferably ammonia or an organic alkali agent.

<22> The hair treatment method according to any one of <1> to <21>, wherein the component (B2) is one or more members selected from the group consisting of ammonia; alkanolamine; alkylamine; and aralkylamine, and the alkanolamine, the alkylamine, or the aralkylamine has preferably 1 or more and 10 or less, more preferably 1 or more and 8 or less carbon atoms.

<23> The hair treatment method according to any one of <1> to <22>, wherein the component (B2) preferably comprises one or more members selected from the group consisting of ammonia, alkanolamine, alkylamine, and aralkylamine, more preferably comprises one or more members selected from the group consisting of ammonia and alkanolamine, and further more preferably comprises alkanolamine.

<24> The hair treatment method according to any one of <1> to <23>, wherein the component (B2) comprises alkanolamine, preferably comprises one or more members selected from the group consisting of monoethanolamine, trishydroxymethylaminomethane, and 2-amino-2-methyl-1-propanol, more preferably comprises one or more members selected from the group consisting of monoethanolamine and 2-amino-2-methyl-1-propanol, and further more preferably comprises monoethanolamine.

<25> The hair treatment method according to <24>, wherein a content of the alkanolamine in the component (B2) is preferably 70% by mass or more, more preferably 80% by mass or more, further more preferably 90% by mass or more, and 100% by mass or less.

<26> The hair treatment method according to any one of <1> to <25>, wherein a content of the component (B2) in the composition B is preferably 0.01% by mass or more, more preferably 0.1% by mass or more, further more preferably 0.5% by mass or more, even more preferably 1.0% by mass or more, even more preferably 2.0% by mass or more, and preferably 10% by mass or less, more preferably 7.5% by mass or less, further more preferably 5.0% by mass or less.

<27> The hair treatment method according to any one of <1> to <26>, wherein the composition B comprises an antioxidant, and the antioxidant is one or more members selected from the group consisting of sulfurous acid, pyrosulfurous acid, hydrogen sulfite, sulfur dioxide, L-ascorbic acid, thioglycolic acid, L-cysteine, N-acetyl-L-cysteine, and a salt thereof, and a derivative thereof, preferably one or more members selected from the group consisting of sulfite, pyrosulfite, hydrosulfite, sulfur dioxide, L-ascorbic acid, and L-ascorbate, more preferably one or more members selected from the group consisting of sodium sulfite and sodium L-ascorbate, further more preferably sodium sulfite.

<28> The hair treatment method according to <27>, wherein a content of the antioxidant is preferably 0.01% by mass or more, more preferably 0.05% by mass or more, further more preferably 0.1% by mass or more, even more preferably 0.2% by mass or more, and preferably 10% by mass or less, more preferably 5.0% by mass or less, further more preferably 3.0% by mass or less, even more preferably 2.0% by mass or less, even more preferably 1.0% by mass or less.

<29> The hair treatment method according to any one of <1> to <28>, wherein the composition B comprises a pH adjuster and preferably a protonating agent as the pH adjuster, and the protonating agent is preferably one or more members selected from the group consisting of a monobasic acid and a polybasic acid, or one or more protonating agents selected from the group consisting of an organic acid (having 1 or more and 8 or less carbon atoms, except for ascorbic acid) and an inorganic acid, more preferably one or more members selected from the group consisting of hydrochloric acid, sulfuric acid, phosphoric acid, formic acid, acetic acid, and citric acid as the protonating agent, further more preferably one or more members selected from the group consisting of phosphoric acid and citric acid.

<30> The hair treatment method according to <29>, wherein a content of the pH adjuster in the composition B is preferably 0.05% by mass or more, more preferably 0.1% by mass or more, further more preferably 0.2% by mass or more, and preferably 5.0% by mass or less, more preferably 4.0% by mass or less, further more preferably 3.5% by mass or less.

<31> The hair treatment method according to any one of <1> to <30>, wherein the composition B comprises a surfactant which is one or more members selected from the group consisting of a cationic surfactant, an anionic surfactant, an amphoteric surfactant, and a nonionic surfactant, and when the composition B is a conditioning agent or a treatment agent, the composition B preferably comprises at least a cationic surfactant as the surfactant.

<32> The hair treatment method according to <31>, wherein a content of the surfactant in the composition B is preferably 0.1% by mass or more, more preferably 0.5% by mass or more, further more preferably 1.0% by mass or more, and preferably 30% by mass or less, more preferably 20% by mass or less.

<33> The hair treatment method according to any one of <1> to <32>, wherein the composition B substantially contains no coloring component, and a content of the coloring component in the composition B is preferably 1% by mass or less, more preferably 0.5% by mass or less, further more preferably 0.2% by mass or less, even more preferably 0% by mass.

<34> The hair treatment method according to any one of <1> to <33>, wherein the composition B substantially contains no oxidizing agent, and a content of the oxidizing agent in the composition B is preferably less than 0.1% by mass, even more preferably less than 0.01% by mass, further more preferably 0% by mass.

<35> The hair treatment method according to any one of <1> to <34>, wherein pH at 25°C of the composition B is preferably 8.0 or higher, more preferably 9.0 or higher, further more preferably 9.5 or higher, even more preferably 9.8 or higher, and preferably 11.0 or lower, more preferably 10.8 or lower, further more preferably 10.5 or lower.

<36> The hair treatment method according to any one of <1> to <35>, wherein a method for applying the composition B to hair is a method of contacting the composition B with the hair, preferably a method of coating hair in a dry state or a wet state with the composition B or a method of immersing hair in a dry state or a wet state in the composition B.

<37> The hair treatment method according to any one of <1> to <36>, wherein an amount of the composition B is a bath ratio (dry mass of the hair : mass of the composition B) of preferably from 1 : 0.1 to 1 : 20, more preferably from 1 : 0.2 to 1 : 10, further more preferably from 1 : 0.3 to 1:5, even more preferably from 1 : 0.4 to 1 : 3.

<38> The hair treatment method according to any one of <1> to <37>, further comprising, after the step (II) of applying the composition B to hair, the step of leaving the resultant to stand, wherein time for which the resultant is left to stand is preferably 1 minute or longer, more preferably 3 minutes or longer, and preferably 30 minutes or shorter, more preferably 20 minutes or shorter.

<39> The hair treatment method according to any one of <1> to <38>, further comprising, after the step (II), the step of rinsing the composition B applied to hair, wherein the rinsing step is preferably performed by washing off the composition B with water, and more preferably a temperature of the water is from 35 to 45°C.

<40> The hair treatment method according to <39>, further comprising, after the hair rinsing step, a drying step of drying the hair, wherein preferably one or more drying treatments selected from the group consisting of towel drying, blow (cold air or hot air) drying, and air drying is performed, and more preferably two or more of the drying treatments are performed in combination.

<41> A method for improving color retention of hair dyed in the following step (I), comprising in order the following step

(I) and step (II):

(I) applying a hair dye composition A comprising the following component (A1) and component (A2) to hair to dye the hair:

(A1) an oxidation dye, and
(A2) hydrogen peroxide; and

(II) applying a composition B comprising the following component (B1) and component (B2) and having pH of 8.0 or higher and 11.0 or lower to the hair:

(B1) a compound of the following formula (1) or a salt thereof:

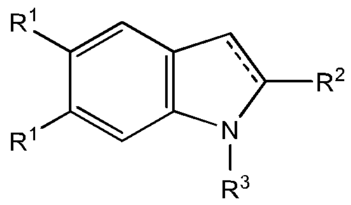

(1)

wherein the broken line represents the presence or absence of a $\pi$ bond; $R^1$ represents a hydroxy group or an acetoxy group; $R^2$ represents a hydrogen atom or -COOR,
wherein R represents a hydrogen atom, a methyl group, or an ethyl group; and $R^3$ represents a hydrogen atom, an acetyl group, a methyl group, or an ethyl group, and

(B2) an alkali agent.

<42> The method for improving color retention according to <41>, having a configuration according to any one of <2> to <40>.
<43> A method for improving color retention of artificially dyed hair, comprising the following step (IIa) :
(IIa) applying a composition B comprising the following component (B1) and component (B2) and having pH of 8.0 or higher and 11.0 or lower to the artificially dyed hair:

(B1) a compound of the following formula (1) or a salt thereof:

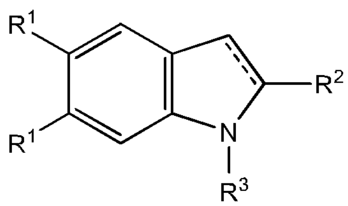

(1)

wherein the broken line represents the presence or absence of a $\pi$ bond; $R^1$ represents a hydroxy group or an acetoxy group; $R^2$ represents a hydrogen atom or -COOR,
wherein R represents a hydrogen atom, a methyl group, or an ethyl group; and $R^3$ represents a hydrogen atom, an acetyl group, a methyl group, or an ethyl group, and

(B2) an alkali agent.

<44> The method for improving color retention according to <43>, comprising a configuration according to any one of <17> to <40>.
(Here, the step (II) is interpreted as a step (IIa).)
<45> A kit for dyeing hair, comprising:

a hair dye composition A comprising the following component (A1) and component (A2):

(A1) an oxidation dye, and
(A2) hydrogen peroxide; and

a composition B comprising the following component (B1) and component (B2) and having pH of 8.0 or higher and 11.0 or lower:

(B1) a compound of the following formula (1) or a salt thereof:

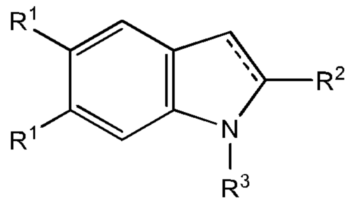

(1)

wherein the broken line represents the presence or absence of a $\pi$ bond; $R^1$ represents a hydroxy group or an acetoxy group; $R^2$ represents a hydrogen atom or -COOR,
wherein R represents a hydrogen atom, a methyl group, or an ethyl group; and $R^3$ represents a hydrogen atom, an acetyl group, a methyl group, or an ethyl group, and

(B2) an alkali agent.

<46> The kit for dyeing hair according to <49>, wherein the hair dye composition A has a configuration according to any one of <2> to <8>.
<47> The kit for dyeing hair according to <45> or <46>, wherein the composition B has a configuration according to any one of <17> to <35>.

Examples

Hereinafter, Examples of the present invention will be described. However, the present invention is not limited by the scope of Examples. In the present Examples, various measurements and evaluations were performed by the following methods.

<pH measurement>

pH at 25°C of a composition B was measured by using a pH meter (F-51, manufactured by HORIBA, Ltd.).

<Lightness ($L_{10}$* value) and hue angle ($h_{10}$ value) of hair dyed in step (I)>

A tress for evaluation mentioned later was used and subjected to a step (I) and a rinsing step in accordance with the methods described in each example. After the rinsing step, the tress was dried in cold air, and an L* value, an a* value, and a b* value were measured. The L* value, the a* value, and the b* value of the tress were measured at six locations per tress using a colorimeter ("CR-400" manufactured by Konica Minolta, Inc.), and averages of the values measured at 6 points were defined as $L_{10}$*, $a_{10}$*, and $b_{10}$*, respectively. A hue angle $h_{10}$ of the tress after the step (I) was calculated in accordance with the following equation and is shown in the tables.

$$\text{Hue angle } h_{10} = \tan^{-1}(b_{10}^* / a_{10}^*)$$

<Color shift $\Delta$h>

A tress for evaluation mentioned later was used and subjected to a step (I) and a rinsing step in accordance with the methods described in Examples 1 to 14. After the rinsing step, the tress was dried in cold air. Then, a* value and a b* value were measured by the method described above, and a hue angle $h_{10}$ of the tress after the step (I) was calculated.
Subsequently, the tress was further subjected to a step (II) and rinsing and drying steps in accordance with the methods described in Examples 1 to 14. An a* value and b* value were measured as to this tress in the same manner as above, and averages of the values measured at 6 points were defined as $a_{20}$* and $b_{20}$*, respectively. A hue angle $h_{20}$ of the

tress after the step (II) was calculated according to the following equation.

$$\text{Hue angle } h_{20} = \tan^{-1}(b_{20}^* / a_{20}^*)$$

A color shift Ah = $h_{20}$ - $h_{10}$ was calculated from $h_{10}$ and $h_{20}$ described above and is shown in the tables. A smaller value of Δh means less discoloration and more favorable results.

<Hair wash resistance ΔL*>

A tress for evaluation mentioned later was used, and the hair tress was treated by the methods of each example.

In Examples 1 to 14, the tress was subjected to a step (I), a rinsing step, a step (II), and rinsing and drying steps by methods mentioned later, followed by L* value measurement. In Comparative Examples other than those described above, the tress was subjected to a step (I) and rinsing and drying steps by methods mentioned later, followed by L* value measurement. The L* value was measured at six locations per tress by using a colorimeter ("CR-400" manufactured by Konica Minolta, Inc.), and an average of the values measured as the L* value at 6 points was defined as $L_{20}$* ($L_{20}$* of Comparative Examples is the same as $L_{10}$* described above).

Subsequently, the tress after the $L_{20}$* measurement was immersed in an aqueous solution composed of a 10-fold dilution of a plain shampoo having the composition given below, and shaken in a warm water bath of 40°C at 120 rpm for 90 minutes. The tress thus shaken was rinsed for 15 seconds and dried in cold air. An L* value of the tress thus dried was measured in the same manner as above, and an average of the values measured at 6 points was defined as $L_{21}$*. A value of $L_{21}$* - $L_2$)* is shown as ΔL* in the tables. A smaller absolute value of ΔL* means better hair wash resistance. In this context, ΔL* of Examples can be expressed as "$\Delta L^*_{\text{after step (I) + (II)}}$", and ΔL* of Comparative Examples can be expressed as "$\Delta L^*_{\text{no step (II)}}$".

| (Plain shampoo) | (% by mass) |
|---|---|
| ▪ Sodium polyoxyethylene lauryl ether sulfate (EMAL E-27C (amount of active ingredient: mass), manufactured by Kao Corp.) | 27% by |
| ▪ Lauramide DEA (AMINON L-02, manufacturedby Kao Corp.) | 57.4 |
| ▪ EDTA-2Na (FROST DS, manufactured by Daiichi Pure Chemicals Co., Ltd.) | 1.5 |
| ▪ Phosphoric acid (for pH adjustment to 7.0) | 0.3 |
| ▪ Sodium benzoate | 0.5 |
| ▪ Purified water | balance |
| Total | 100 |

<Degree of improvement in hair wash resistance>

A tress for evaluation mentioned later was used and, in Examples 1 to 14, subjected to step (I), rinsing, and drying in accordance with the methods described in each example. Subsequently, the tress was immersed in an aqueous solution composed of a 10-fold dilution of a plain shampoo having the composition described above, and shaken in a warm water bath of 40°C at 120 rpm for 90 minutes. The tress thus shaken was rinsed for 15 seconds and dried in cold air. An L* value of the tress thus dried was measured in the same manner as above, and an average of the values measured at 6 points was defined as $L_{11}$*.

In this context, a lightness of the hair before hair wash is $L_{10}$* described above, and a value of $L_{11}$* - $L_{10}$* represents the hair wash resistance of the hair subjected to the step (I). This is the same as the hair wash resistance "$\Delta L^*_{\text{no step (II)}}$". The hair wash resistance ΔL* of Examples is "$\Delta L^*_{\text{after step (I) + (II)}}$", as described above.

A delta of absolute values thereof ($|\Delta L^*_{\text{after step (I) + (II)}}| - |\Delta L^*_{\text{no step (II)}}|$) was calculated, and this value is shown as the degree of improvement in hair wash resistance in the tables. A larger value thereof means a better effect of improving hair wash resistance.

Example 1 (hair treatment and evaluation)

<Preparing tress for evaluation>

An untreated hair tress (gray hair tress of a Chinese female having a length of 10 cm and a mass of 1 g, "BM-W-A" manufactured by Beaulax Co., Ltd.,) was cleansed once with a plain shampoo having the composition described above,

blow-dried with hot air of a dryer for 30 minutes, and left to stand overnight or longer at a temperature of 25°C under a relative humidity of 65% to prepare a hair tress for evaluation.

<Step (I)>

A hair color 1 ("Topchic6N" manufactured by Goldwell) was used as a hair dye composition A. The hair color 1 was prepared as described in the product instruction, and the tress for evaluation was coated with the hair color at a bath ratio (mass of the hair in a dry state : mass of the hair color 1) of 1 : 2 and left to stand for 20 minutes in an atmosphere of 40°C.

<Rinsing step>

After a lapse of 20 minutes, the hair color 1 was washed off with running warm water (approximately 40°C) for 30 seconds. After hair wash with the plain shampoo for 15 seconds, the shampoo was washed off with running warm water (approximately 40°C) for 15 seconds. This tress was subjected to the following step (II).

<Step (II)>

A component (B1), a mixture of 5,6-dihydroxy indole and 5,6-dihydroxyindole-2-carboxylic acid (which will be mentioned hereinafter in detail) was blended in the amount shown in Table 1 with and dissolved in an aqueous solution containing 3% by mass of monoethanolamine in a nitrogen atmosphere to prepare a liquid composition B. In the description below, all the amounts of components blended described in the tables are % by mass of the amount of an active ingredient. The prepared composition B was stored in a nitrogen atmosphere, and an aliquot was separated therefrom upon application in each case.

The tress thus towel-dried was coated with the composition B in an amount which attained a bath ratio (mass of the hair in a dry state : mass of the composition B) = 1 : 0.5 and left to stand in atmosphere for 5 minutes in this state.

<Rinsing and drying steps>

After a lapse of 5 minutes, the tress was washed with running warm water (approximately 40°C) for 30 seconds. The tress was towel-dried and subsequently blow-dried with hot air of a dryer for 30 minutes.

Table 1 shows results of performing various evaluations by the methods described above by using the tress thus treated.

Examples 2 to 14

A hair treatment and evaluations were performed by the same methods as in Example 1 except that the hair color and the composition B used were changed to those shown in Table 1 or 2. The results are shown in Table 1 or 2.

Comparative Examples 1 to 9

A hair treatment and evaluations were performed by the same methods as in Example 1 except that the hair color shown in Table 1 or 2 was used in the step (I); and the subsequent rinsing step and step (II) were not performed. The results are shown in Table 1 or 2.

[Table 1]

Table 1

| | | | $L_{10}^*$ | $h_{10}$ | Example | | | | | | | Comparative Example | | | | | | |
|---|---|---|---|---|---|---|---|---|---|---|---|---|---|---|---|---|---|---|
| | | | | | 1 | 2 | 3 | 4 | 5 | 6 | 7 | 1 | 2 | 3 | 4 | 5 | 6 | 7 |
| Step (I) | Hair dye composition A | Hair color 1 | 28.09 | 54.95 | ○ | | | | | | | ○ | | | | | | |
| | | Hair color 2 | 30.35 | 7.36 | | ○ | | | | | | | ○ | | | | | |
| | | Hair color 3 | 30.48 | 46.66 | | | ○ | | | | | | | ○ | | | | |
| | | Hair color 4 | 29.17 | 39.44 | | | | ○ | | | | | | | ○ | | | |
| | | Hair color 5 | 41.73 | 67.25 | | | | | ○ | | | | | | | ○ | | |
| | | Hair color 6 | 43.25 | 71.28 | | | | | | ○ | | | | | | | ○ | |
| | | Hair color 7 | 47.03 | 59.05 | | | | | | | ○ | | | | | | | ○ |
| Step (II) | Composition B | Mixture of 5,6-dihydroxyindole and 5,6-dihydroxyindole-2-carboxylic acid | % by mass | | 0.075 | 0.075 | 0.075 | 0.075 | 0.075 | 0.075 | 0.075 | - | - | - | - | - | - | - |
| | | Monoethanolamine | % by mass | | 3 | 3 | 3 | 3 | 3 | 3 | 3 | - | - | - | - | - | - | - |
| | | Pure water | % by mass | | Balance | | | | | | | - | - | - | - | - | - | - |
| | | 75% phosphoric acid | % by mass | | q.s. (pH = 10.2) | | | | | | | - | - | - | - | - | - | - |
| | | Total | % by mass | | 100 | 100 | 100 | 100 | 100 | 100 | 100 | - | - | - | - | - | - | - |
| | | pH (25°C) | - | | 10.2 | 10.2 | 10.2 | 10.2 | 10.2 | 10.2 | 10.2 | - | - | - | - | - | - | - |
| | Bath ratio (hair : composition B) | | - | | 1 : 0.5 | 1 : 0.5 | 1 : 0.5 | 1 : 0.5 | 1 : 0.5 | 1 : 0.5 | 1 : 0.5 | - | - | - | - | - | - | - |
| | Time for which sample was left to stand | | min | | 5 | 5 | 5 | 5 | 5 | 5 | 5 | - | - | - | - | - | - | - |
| Evaluation results | Color shift $\Delta h$ | | - | | -0.20 | 1.33 | 0.41 | 0.03 | 1.50 | 0.73 | -2.63 | - | - | - | - | - | - | - |
| | Hair wash resistance $\Delta L^*$ | | - | | 3.90 | 4.34 | 2.44 | 3.06 | 1.11 | -0.43 | 5.31 | 5.19 | 8.24 | 5.07 | 7.08 | 3.71 | 4.10 | 7.49 |
| | Degree of improvement in hair wash: $\lvert \Delta L^*_{no\ step\ (II)} \rvert - \lvert \Delta L^*_{after\ step\ (I) + (II)} \rvert$ | | - | | 1.29 | 3.90 | 2.63 | 4.02 | 2.60 | 3.67 | 2.18 | - | - | - | - | - | - | - |

[Table 2]

Table 2

| | | | $L_{10}^*$ | $h_{10}$ | Example | | | | | | | Comparative Example | | | | | |
|---|---|---|---|---|---|---|---|---|---|---|---|---|---|---|---|---|---|
| | | | | | 8 | 9 | 10 | 11 | 12 | 13 | 14 | 8 | 2 | 3 | 9 | 5 | 7 |
| Step (I) | Hair dye composition A | Hair color 8 | 24.00 | 47.50 | ○ | | | | | | ○ | ○ | | | | | |
| | | Hair color 2 | 30.35 | 7.36 | | ○ | | | | | | | ○ | | | | |
| | | Hair color 3 | 30.48 | 46.66 | | | ○ | | | | | | | ○ | | | |
| | | Hair color 9 | 35.98 | 51.12 | | | | ○ | | | | | | | ○ | | |
| | | Hair color 5 | 41.73 | 67.25 | | | | | ○ | | | | | | | ○ | |
| | | Hair color 7 | 47.03 | 59.05 | | | | | | ○ | | | | | | | ○ |
| Step (II) | Composition B | Mixture of 5,6-dihydroxyindole and 5,6-dihydroxyindole-2-carboxylic acid | % by mass | | 0.05 | 0.25 | 0.25 | 0.25 | 0.25 | 0.25 | 0.5 | - | - | - | - | - | - |
| | | Monoethanolamine | % by mass | | 3 | 3 | 3 | 3 | 3 | 3 | 3 | - | - | - | - | - | - |
| | | Pure water | % by mass | | Balance | | | | | | | - | - | - | - | - | - |
| | | 75% phosphoric acid | % by mass | | q.s. (pH = 10.2) | | | | | | | - | - | - | - | - | - |
| | | Total | % by mass | | 100 | 100 | 100 | 100 | 100 | 100 | 100 | - | - | - | - | - | - |
| | | pH (25°C) | - | | 10.2 | 10.2 | 10.2 | 10.2 | 10.2 | 10.2 | 10.2 | - | - | - | - | - | - |
| | Bath ratio (hair : composition B) | | - | | 1 : 0.5 | 1 : 0.5 | 1 : 0.5 | 1 : 0.5 | 1 : 0.5 | 1 : 0.5 | 1 : 0.5 | - | - | - | - | - | - |
| | Time for which sample was left to stand | | min | | 5 | 5 | 5 | 5 | 5 | 5 | 5 | - | - | - | - | - | - |
| Evaluation results | Color shift Δh | | - | | -0.86 | -0.16 | 3.51 | 2.21 | 4.75 | -7.65 | -4.32 | - | - | - | - | - | - |
| | Hair wash resistance ΔL* | | - | | 3.98 | 3.22 | 2.29 | 5.16 | -0.42 | 2.55 | 1.67 | 6.90 | 8.24 | 5.07 | 8.51 | 3.71 | 7.49 |
| | Degree of improvement in hair wash: $\lvert\Delta L^*_{\text{no step (II)}}\rvert - \lvert\Delta L^*_{\text{after step (I) +(II)}}\rvert$ | | - | | 2.92 | 5.02 | 2.78 | 3.35 | 3.29 | 4.94 | 5.23 | - | - | - | - | - | - |

The following was used as the hair dye composition A and the component (B1) described in the tables. The content of the oxidation dye in the hair dye composition A was from 0.1 to 2% by mass, and the content of the hydrogen peroxide was from 3.0 to 3.6% by mass. The hair color 4 was in a foam, and the other hair colors were in a cream.

<Hair dye composition A>

- Hair color 1: "Topchic6N" manufactured by Goldwell
- Hair color 2: "Topchic6BP" manufactured by Goldwell
- Hair color 3: "Topchic6G" manufactured by Goldwell
- Hair color 4: "Blaune Foam Color 1PK" manufactured by Kao Corp.
- Hair color 5: "Topchic8N" manufactured by Goldwell
- Hair color 6: "Topchic8A" manufactured by Goldwell
- Hair color 7: "Blaune Lumiest 0B" manufactured by Kao Corp.
- Hair color 8: "Newance by Topchic 8CB" manufactured by Kao Salon Japan Co., Ltd.
- Hair color 9: "Topchic6K" manufactured by Goldwell

<Component (B1)>

▪ Mixture of 5,6-dihydroxyindole and 5,6-dihydroxyindole-2-carboxylic acid: produced by the method described in JP-B-5570161 for use. The molar ratio of the 5,6-dihydroxyindole and the 5,6-dihydroxyindole-2-carboxylic acid was 90 : 10. The component (B1) was prepared as a solution containing 1% by mass thereof in $H_2O$/ethanol = 4/1 (wt/wt), which was blended with composition B.

Industrial Applicability

The present invention provides a hair treatment method which offers less discoloration of hair after dyeing and improves color retention.

## Claims

**1.** A hair treatment method comprising in order the following step (I) and step (II):

(I) applying a hair dye composition A comprising the following component (A1) and component (A2) to hair to dye the hair:

(A1) an oxidation dye, and
(A2) hydrogen peroxide; and

(II) applying a composition B comprising the following component (B1) and component (B2) and having pH of 8.0 or higher and 11.0 or lower to the hair:

(B1) a compound of the following formula (1) or a salt thereof:

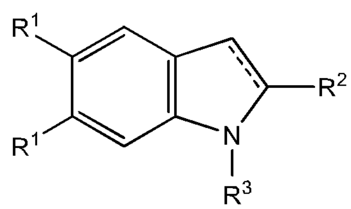

(1)

wherein the broken line represents the presence or absence of a $\pi$ bond; $R^1$ represents a hydroxy group or an acetoxy group; $R^2$ represents a hydrogen atom or -COOR,
wherein R represents a hydrogen atom, a methyl group, or an ethyl group; and $R^3$ represents a hydrogen atom, an acetyl group, a methyl group, or an ethyl group, and

(B2) an alkali agent.

**2.** The treatment method according to claim 1, wherein a content of the component (B1) in the composition B is 0.01% by mass or more and 3.0% by mass or less.

**3.** The treatment method according to claim 1 or 2, wherein a content of the component (B2) in the composition B is 0.01% by mass or more and 10% by mass or less.

**4.** The treatment method according to any one of claims 1 to 3, further comprising, between the step (I) and the step (II), the step of washing off the hair dye composition A applied to the hair.

**5.** A method for improving color retention of hair dyed in the following step (I), comprising in order the following step (I) and step (II):

(I) applying a hair dye composition A comprising the following component (A1) and component (A2) to hair to dye the hair:

(A1) an oxidation dye, and
(A2) hydrogen peroxide; and

(II) applying a composition B comprising the following component (B1) and component (B2) and having pH of 8.0 or higher and 11.0 or lower to the hair:

(B1) a compound of the following formula (1) or a salt thereof:

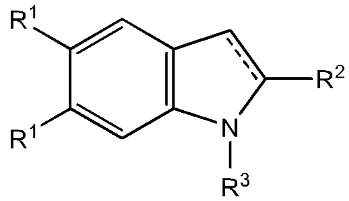

(1)

wherein the broken line represents the presence or absence of a $\pi$ bond; $R^1$ represents a hydroxy group or an acetoxy group; $R^2$ represents a hydrogen atom or -COOR,
wherein R represents a hydrogen atom, a methyl group, or an ethyl group; and $R^3$ represents a hydrogen atom, an acetyl group, a methyl group, or an ethyl group, and

(B2) an alkali agent.

**6.** A method for improving color retention of artificially dyed hair, comprising the following step (IIa) :
(IIa) applying a composition B comprising the following component (B1) and component (B2) and having pH of 8.0 or higher and 11.0 or lower to the artificially dyed hair:

(B1) a compound of the following formula (1) or a salt thereof:

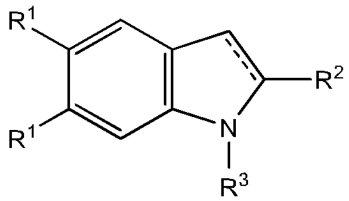

(1)

wherein the broken line represents the presence or absence of a $\pi$ bond; $R^1$ represents a hydroxy group or an acetoxy group; $R^2$ represents a hydrogen atom or -COOR,
wherein R represents a hydrogen atom, a methyl group, or an ethyl group; and $R^3$ represents a hydrogen atom, an acetyl group, a methyl group, or an ethyl group, and

(B2) an alkali agent.

**7.** A kit for dyeing hair, comprising:

a hair dye composition A comprising the following component (A1) and component (A2):

(A1) an oxidation dye, and
(A2) hydrogen peroxide; and

a composition B comprising the following component (B1) and component (B2) and having pH of 8.0 or higher and 11.0 or lower:

(B1) a compound of the following formula (1) or a salt thereof:

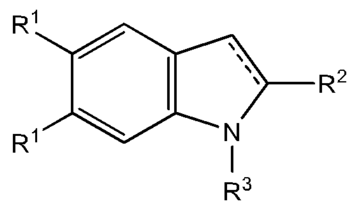

(1)

wherein the broken line represents the presence or absence of a $\pi$ bond; $R^1$ represents a hydroxy group or an acetoxy group; $R^2$ represents a hydrogen atom or -COOR, wherein R represents a hydrogen atom, a methyl group, or an ethyl group; and $R^3$ represents a hydrogen atom, an acetyl group, a methyl group, or an ethyl group, and

(B2) an alkali agent.

**INTERNATIONAL SEARCH REPORT**

International application No.
**PCT/JP2024/002635**

**A. CLASSIFICATION OF SUBJECT MATTER**

***A61K 8/49***(2006.01)i; ***A61K 8/22***(2006.01)i; ***A61K 8/41***(2006.01)i; ***A61Q 5/06***(2006.01)i
FI: A61K8/49; A61K8/41; A61Q5/06; A61K8/22

According to International Patent Classification (IPC) or to both national classification and IPC

**B. FIELDS SEARCHED**

Minimum documentation searched (classification system followed by classification symbols)

A61K8/49; A61K8/22; A61K8/41; A61Q5/06

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY/MEDLINE/EMBASE/BIOSIS/KOSMET (STN); Mintel GNPD

**C. DOCUMENTS CONSIDERED TO BE RELEVANT**

| Category* | Citation of document, with indication, where appropriate, of the relevant passages | Relevant to claim No. |
|---|---|---|
| A | JP 6-135824 A (BRISTOL-MYERS SQUIBB COMPANY) 17 May 1994 (1994-05-17) entire text | 1-7 |
| A | JP 4-227974 A (L'OREAL) 18 August 1992 (1992-08-18) entire text | 1-7 |
| A | JP 7-508271 A (L'OREAL) 14 September 1995 (1995-09-14) entire text | 1-7 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 April 2024** | **16 April 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/002635**

| Patent document cited in search report | Publication date (day/month/year) | Patent family member(s) | Publication date (day/month/year) |
|---|---|---|---|
| JP 6-135824 A | 17 May 1994 | US 5173085 A<br>entire text<br>GB 2132642 A<br>KR 10-1989-0000189 A | |
| JP 4-227974 A | 18 August 1992 | US 5178637 A<br>entire text<br>EP 462857 A1<br>CN 1047592 A<br>KR 10-1991-0019595 A | |
| JP 7-508271 A | 14 September 1995 | US 5538317 A<br>entire text<br>WO 1994/000100 A1<br>EP 645999 A1 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2015140326 A
- JP 2005170838 A
- JP 2005320355 A
- JP 2008133227 A
- JP 2021059517 A
- JP 2021123535 A
- JP 2003342139 A
- JP 5570161 B